# EUROPÄISCHE PATENTSCHRIFT

(11) **EP 2 589 542 B1**
(45) Veröffentlichungstag und Bekanntmachung des Hinweises auf die Patenterteilung: **14.06.2017**
(21) Anmeldenummer: 12191094.7
(22) Anmeldetag: 02.11.2012
(51) Int. Cl.: B65B 55/02, B67C 7/00, A61L 2/08, H01J 37/30, H01J 33/02

(54) **Vorrichtung zum Sterilisieren von Kunststoffbehältnissen mittels mediengesteuerter Elektronenstrahlen**
Device for sterilising plastic containers by means of media-controlled electron beams
Dispositif pour la stérilisation de récipients en plastique au moyen d'un rayonnement à électrons dirigé par un milieu

(30) Priorität: 02.11.2011 DE 102011055005
(43) Veröffentlichungstag der Anmeldung: 08.05.2013
(73) Patentinhaber: Krones AG, 93073 Neutraubling (DE)
(72) Erfinder: Knott, Josef, 93073 Neutraubling (DE); Scheuren, Hans, 93073 Neutraubling (DE)
(74) Vertreter: Bittner, Bernhard

(56) Entgegenhaltungen:
- EP-A1- 1 982 920
- EP-A1- 2 103 528
- WO-A1-97/07024
- WO-A1-02/058742
- WO-A1-2008/073015

## Beschreibung

Die Erfindung betrifft eine Vorrichtung zum Sterilisieren von einer Innenwandung von Behältnissen mittels beschleunigter Ladungsträger mit einer Ladungsträgerquelle zum Erzeugen der Ladungsträger, mit einer Beschleunigungseinrichtung, durch welche Ladungsträger in Richtung eines Ladungsträgeraustrittsfensters beschleunigbar sind, wobei das Ladungsträgeraustrittsfenster an einer Behandlungseinrichtung angeordnet ist, welche entlang einer Einführrichtung durch eine Öffnung in das Behältnis einführbar ist, um eine Innenwandung des Behältnisses mit den aus dem Ladungsträgeraustrittsfenster austretenden Ladungsträgern zu beaufschlagen, wobei die Behandlungseinrichtung eine Medienleitung mit einer Medienaustrittsöffnung aufweist, deren Medienaustrittsöffnung bevorzugt durch die Öffnung in das Behältnis einführbar ist.

Weiterhin betrifft die Erfindung eine Anlage zum Behandeln von Behältnissen, welche mindestens eine Vorrichtung zum Sterilisieren von Behältnissen aufweist, sowie ein Verfahren zum Sterilisieren von Behältnissen und insbesondere einer Innenwandung von Behältnissen mittels beschleunigter Ladungsträger, wobei Ladungsträger in einer Ladungsträgerquelle erzeugt werden und mit einer Beschleunigungseinrichtung in Richtung eines Ladungsträgeraustrittsfensters beschleunigt werden, wobei das Ladungsträgeraustrittsfenster an einer Behandlungseinrichtung angeordnet ist, welche entlang einer Einführrichtung durch eine Öffnung in das Behältnis eingeführt wird, um eine Innenwandung des Behältnisses mit den aus dem Ladungsträgeraustrittsfenster austretenden Ladungsträgern zu beaufschlagen, wobei die Behandlungseinrichtung eine Medienleitung mit einer Medienaustrittsöffnung aufweist, deren Medienaustrittsöffnung durch die Öffnung in das Behältnis einführbar ist.

Die Sterilisation eines zu befüllenden Behälters ist neben dem eigentlichen Füllvorgang der zentrale Prozessschritt in einer aseptischen Abfüllanlage. Die möglichen Sterilisationsformen variieren hinsichtlich der Entkeimungsmittel und der Prozessführung. Allen gemein ist jedoch, dass die abtötende Wirkung aufgrund chemischer Prozesse bewirkt wird. So ist es beispielsweise bekannt, die Innenwandung von Behältnissen mit Dampf oder Wasserstoffperoxid zu sterilisieren. Derartige Verfahren sind jedoch mit Nachteilen verbunden, da es durch die Behandlung beispielsweise mit Wasserstoffperoxid zu einer Aufweichung des Materials kommen kann. Neuere Entwicklungen grenzen sich davon ab und nutzen ionisierende Strahlung um eine Keimreduzierung zu erreichen. Diese Strahlung besteht in den meisten Anwendungen aus beschleunigten Elektronen, die in einer entsprechenden Anlage erzeugt und in den zu sterilisierenden Behälter eingeführt werden. Daraus ergibt sich eine Verringerung oder vollständige Vermeidung des Einsatzes chemischer Stoffe und unter anderem eine Reduzierung der Beschaffungs- und Entsorgungskosten.

Die Begriffe "Behältnis", "Behälter" und "Kunststoffbehältnis" werden im Folgenden zur Vereinfachung synonym verwendet. Dabei schließen diese Begriffe auch Vorprodukte derartiger Behältnisse ein. Insbesondere beziehen sich diese Begriffe auf Flaschen, bevorzugt Getränkeflaschen aber auch auf Vorformlinge (z.B. Kunststoffvorformlinge für z.B. Flaschen).

Entsprechende aus dem Stand der Technik bekannte Systeme zur Sterilisation, bestehen aus einer Elektronenerzeugungsvorrichtung und einer Bündelungseinrichtung. Außerhalb des zu sterilisierenden Behältnisses erzeugte Ladungsträger werden durch verschiedene z.B. mechanische oder elektronische Elemente in das zu sterilisierende Behältnis gelenkt. In diesem bildet sich eine Wolke aus Elektronen welche durch Wechselwirkungen mit etwaigen unerwünschten Mikroorganismen diese inaktiviert.

So beschreibt die DE 198 82 252 T1 eine Technik zur Innensterilisation eines Behälters mittels Elektronen. Dabei ist ebenfalls eine Elektronenstrahlquelle vorgesehen, die Strahlung von außerhalb in das Innere des Behältnisses richtet.

Wie beispielsweise aus Druckschrift WO 97/07024 A sind auch Verfahren bekannt, in denen zumindest Teile der Elektronenquelle in das Behältnisinnere eingeführt werden können. Es ist ein Verfahren zum Reinigung bzw. Sterilisieren von Produktverpackungen mittels einer Kombination aus Elektronenstrahl und Gasstrahl beschrieben. Die in WO 97/07024 A offenbarte Vorrichtung umfasst eine teilweise in das Behältnisinnere einführbare Elektronenkanone welche beschleunigte Elektronen in das Innere eines Behältnisses einbringt. Ein parallel dazu eingeleiteter Strom eines mit dem Elektronenstrahl wechselwirkenden Gases dient entweder zur Ablenkung des Elektronenstrahls in Richtung des Gasstroms oder aber durch Ionisierung des Gases als Hilfsstoff zu Sterilisierung.

Auch sind den Druckschriften WO 97/07024 und EP 1982920 A1 Vorrichtungen zum Sterilisieren von Behältnissen bekannt.

Die WO 02/058742 A1 zeigt ein Verfahren zum Sterilisieren von Oberflächen lebender Kreaturen.

In vielen Fällen ist es jedoch auch wünschenswert, die Geometrie der Elektronenwolke zu verändern, insbesondere deren Ausdehnung zu verringern. Ein Vorteil eines Prozesses mit gesteuerter Verkleinerung der Elektronenwolke besteht darin, dass die Strahlerleistung und damit auch die Dimension der entstehenden Wolke ohne Änderung der Strahlereinstellung, z.B. der Beschleunigungsspannung, an beispielsweise verkleinerte Behälter, wie etwa Behältnisvorformlinge (Preforms) angepasst werden könnte. Zu stark beschleunigte Elektronen könnten zu tief in das Preformmaterial eindringen, ohne ausreichend mit der Oberfläche und darauf befindlichen Verunreinigungen wechselwirken zu können und somit die Oberfläche zu sterilisieren. Weiterhin könnten zu stark beschleunigte Ladungsträger - je nach verwendetem Behältnismaterial - evtl. auch das Behältnismaterial beschädigen.

Besonders vorteilhaft wäre es, wenn die Ausdehnung der Elektronenwolke bis in einen Bereich verringert werden könnte, in welchem die von der Behandlungseinrichtung austretende Strahlung, welche sowohl den Ladungsträgerstrahl als auch Störstrahlung, z.B. Röntgenstrahlung einschließen kann, gegenüber der Umgebung weitgehend abgeschirmt werden kann und so bei einem Entfernen der Behandlungseinrichtung aus dem zu sterilisierenden Behältnis, eine unnötige Strahlenbelastung der Umgebung vermieden werden kann. Dies könnte z.B. bei einem Wechsel der zu sterilisierenden Behältnisse vorteilhaft sein, bei welchem die Behandlungseinrichtung und somit auch die abgegebene Elektronenwolke kurzzeitig außerhalb eines Behältnisses sind.

Der Erfindung liegt somit die Aufgabe zugrunde, eine Vorrichtung zum Sterilisieren von Behältnissen bereitzustellen, welche es ermöglicht, ohne Abschaltung oder Änderung der Beschleunigungsspannung die Dimension einer von einer Behandlungseinrichtung abgestrahlten Elektronenwolke zu verändern und insbesondere zu verringern.

Da insbesondere in den zur Produktion und Befüllung von Behältnissen verwendeten Anlagen hohe Taktraten und Durchsatzzahlen erreicht werden, ist es weiterhin Aufgabe der Erfindung eine entsprechende Anlage zum Behandeln von Behältnissen bereitzustellen, welche eine derartige Vorrichtung zum Sterilisieren von Behältnissen einschließt.

Darüber hinaus ist es Aufgabe der Erfindung, ein Verfahren zum Sterilisieren von Behältnissen bereitzustellen, welches es ermöglicht, ohne Abschaltung oder Änderung der Beschleunigungsspannung die Dimension einer von einer Behandlungseinrichtung abgestrahlten Elektronenwolke zu verändern und insbesondere zu verringern.

Dies wird erfindungsgemäß durch eine Vorrichtung nach Anspruch 1, eine Anlage nach Anspruch 4 und ein Verfahren nach Anspruch 6 erreicht. Vorteilhafte Ausführungsformen und Weiterbildungen sind Gegenstand der Unteransprüche.

Ein wesentlicher Aspekt der Erfindung ist eine Vorrichtung zum Sterilisieren von Behältnissen und insbesondere einer Innenwandung von Behältnissen mittels beschleunigter Ladungsträger mit einer Ladungsträgerquelle zum Erzeugen der Ladungsträger, mit einer Beschleunigungseinrichtung, durch welche Ladungsträger in Richtung eines Ladungsträgeraustrittsfensters beschleunigbar sind, wobei das Ladungsträgeraustrittsfenster an einer Behandlungseinrichtung angeordnet ist, welche entlang einer Einführrichtung durch eine Öffnung in das Behältnis einführbar ist, um eine Innenwandung des Behältnisses mit den aus dem Ladungsträgeraustrittsfenster austretenden Ladungsträgern zu beaufschlagen, wobei die Behandlungseinrichtung eine Medienleitung mit einer Medienaustrittsöffnung aufweist, deren Medienaustrittsöffnung bevorzugt durch die Öffnung in das Behältnis einführbar ist, wobei die Behandlungseinrichtung mindestens einen in Einführrichtung gegenüber der Ladungsträgeraustrittsfenster vorstehenden Vorsprung aufweist, welcher dazu geeignet ist, Störstrahlung zu absorbieren und wobei durch die Medienleitung ein Medium führbar ist, welches durch die Medienaustrittsöffnung zumindest in den Bereich der aus der Ladungsträgeraustrittsfenster austretenden Ladungsträger abgebbar ist, wobei das Medium dazu geeignet ist, die Dimension einer durch die ausgetretenen Ladungsträger gebildeten Ladungsträgerwolke zu verändern.

Mit einer derartigen Vorrichtung ist es möglich, die Dimension der durch die ausgetretenen Ladungsträger gebildeten Ladungsträgerwolke ohne eine Veränderung der Parameter, welche die Entstehung und Beschleunigung der Ladungsträger vor dessen Durchtritt durch das Ladungsträgeraustrittsfensters beeinflussen, zu verkleinern oder zu vergrößern. Insbesondere ist es unabhängig von der Leistung des Strahlers möglich, die Geometrie der Elektronenwolke, welche ein entscheidendes Kriterium für den Sterilisationserfolg darstellt, zu beeinflussen. Der Vorteil einer solchen Prozesskontrolle besteht darin, ohne Änderung der Strahlereinstellung die entstehende Ladungsträgerwolke an beispielsweise verkleinerte Behälter, wie etwa Preforms anpassen zu können. Beispielsweise ist es möglich, durch eine solche Vorrichtung möglich, eine eingestellte Strahlerleistung beizubehalten, auch wenn der Sterilisationsprozess umgestellt wird und ein Wechsel von einem großen Behältnis mit einem Volumen von z.B. 1 ,5l auf ein kleineres Behältnis wie z.B. einen Vorformling (Preform) mit dementsprechend kleinerem Volumen und geringerer Oberfläche vorgenommen wird. Für eine solche Preform wäre die für die Sterilisierung des 1 ,5I-Behältnisses geeignete Beschleunigung der Ladungsträger ohne nachträgliche Modulation zu stark und die Ladungsträger würden somit zu tief in das Preformmaterial selbst eindringen, ohne die Oberfläche ausreichend zu sterilisieren, oder sogar das Preformmaterial dauerhaft beschädigen.

Der Querschnitt der Behandlungseinrichtung ist derart beschaffen, dass die Behandlungseinrichtung zumindest teilweise, bevorzugt mit dem Behandlungskopf, durch die Mündung des Behältnisses führbar ist.

Die Beschleunigungseinrichtung beschleunigt die Ladungsträger derart, dass die aus dem Austrittsfenster austretenden Ladungsträger bevorzugt direkt auf eine Innenwandung des Behältnisses richtbar sind.

Bei den Ladungsträgern handelt es sich insbesondere um Elektronen, es wäre jedoch auch denkbar, dass andere Ladungsträger, wie Ionen, verwendet werden.

Das Ladungsträgeraustrittsfenster ist besonders bevorzugt aus einem Material hergestellt, welches aus einer Gruppe von Materialien ausgewählt ist, welche Titan, Quarzglas, Diamant, Kombinationen hieraus und dergleichen enthält.

Bei der erfindungsgemäßen Vorrichtung ist es möglich, durch die Medienleitung ein Medium zu führen, welches die Ladungsträger nach deren Durchtritt durch das Ladungsträgeraustrittsfensters beeinflusst. Insbesondere ist es möglich, Ladungsträger abzubremsen und/oder diese in ihrer (Beschleunigungs-) Energie bzw. ihrem Impuls zu reduzieren. Dies kann beispielsweise durch Einleiten eines schweren Gases oder eines anderen Mediums erfolgen und so die gewünschte Wirkung an der Preforminnenwandung erreicht werden. Als schweres Gas werden diesbezüglich insbesondere Gase eines großen Wirkungsquerschnitts, großen Stoßquerschnitts, einer hohen Dichte und/oder starker Wechselwirkung mit Ladungsträgern verstanden. Besonders vorteilhaft sind Medien, welche bei der kinetischen Energie der Ladungsträger einen großen Wirkungsquerschnitts oder Stoßquerschnitt aufweisen.

Ebenso ist es denkbar, den entgegengesetzten Fall zu verwirklichen. So kann eine vergleichsweise voreingestellte Beschleunigungsspannung und damit z.B. die kinetische Energie der Ladungsträger bei einem Behältniswechsel zu gering für eine ausreichende Sterilisierung der (z.B. inneren) Behältnisoberfläche sein. Dies kann z.B. bei einem Wechsel von einem kleinen auf ein größeres Behältnis (größerer Behälter) vorkommen. Durch Einleiten eines entsprechenden Mediums, z.B. leichten Gases, kann die Reichweite der sterilisierenden Ladungsträger (z.B. Elektronen) bei gegebener Beschleunigungsspannung erhöht werden. Als leichtes Gas werden im Folgenden insbesondere Gase eines geringen Wirkungsquerschnitts, geringen Stoßquerschnitts einer geringen Dichte und/oder schwachen Wechselwirkung mit Gasmolekülen verstanden. Durch das Einführen eines derartigen Mediums kann ein in dem Bereich der Ladungsträgerwolke evtl. vorhandenes Medium mit einem höherem Wirkungsquerschnitt, höherem Stoßquerschnitt, einer höheren Dichte und/oder stärker Wechselwirkung mit Ladungsträgern verdrängt werden und die Reichweite der Ladungsträger erhöht werden.

Eine weitere Möglichkeit zur Einflussnahme auf die Dimension der Ladungsträgerwolke besteht in der Erhöhung oder Verringerung des Kammerdrucks, z.B. des Gasdrucks. Hierbei ist insbesondere eine Veränderung des Drucks, bevorzugt des Gasdrucks im Bereich der Ladungsträgerwolke vorteilhaft. Die Wirkungsweise entspricht der der beschriebenen Verfahren zur Dichtevariation.

Eine weiterer wesentlicher Aspekt der Erfindung einer Vorrichtung zum Sterilisieren von Behältnissen ist, dass die Behandlungseinrichtung mindestens einen in Einführrichtung gegenüber dem Ladungsträgeraustrittsfenster vorstehenden Vorsprung aufweist, welcher dazu geeignet ist, Störstrahlung zu absorbieren. Dieser Vorsprung ist in der Nähe des Ladungsträgeraustrittsfensters angeordnet und erstreckt sich von einer durch das Ladungsträgeraustrittsfenster gebildeten Ebene mindestens auch in Richtung des austretenden Ladungsträgerstrahls, und weist bevorzugt eine (Vektor-) Komponente in dieser Richtung auf. Bevorzugt ist der Vorsprung senkrecht zur der durch das Ladungsträgeraustrittsfenster gebildeten Ebene angeordnet. Es sind jedoch auch Ausführungen möglich, in welcher der Vorsprung um einen Winkel bis zu ± 10°, ± 20°, ± 30°oder sogar bis ± 45° von der Senkrechten abweicht. Besonders sind Winkel des Vorsprungs bevorzugt, bei welchen das vom Ladungsträgeraustrittsfenster entfernte Ende des Vorsprungs in Richtung einer zentralen Längsachse der Behandlungseinrichtung, bevorzugt einer durch das Zentrum des Ladungsträgeraustrittsfensters verlaufenden Achse, geneigt ist, so dass auf den Vorsprung treffende Störstrahlung bevorzugt in Richtung des Ladungsträgeraustrittsfensters reflektiert wird.

Dies bietet zusätzlich auch Vorteile beim Einführen in das Behältnis, da dadurch eine kegelstumpfartige Form der Spitze der Behandlungseinrichtung entsteht, welche auch dann, falls eine zentrale Achse des Behältnisses oder dessen Öffnung gegenüber einer zentralen Achse der Behandlungseinrichtung leicht versetzt angeordnet sein sollte, noch ohne Beschädigungen des Behältnisses oder der Spitze der Behandlungseinrichtung in das Behältnis einführbar ist.

Der Vorsprung dient dazu, Störstrahlung und/oder Elektronen zu absorbieren. Störstrahlung tritt insbesondere in der Nähe der Ladungsträgererzeugung z.B. in Form von Röntgenstrahlung auf. Jedoch sind auch andere Arten von Störstrahlung möglich. Insbesondere kann Störstrahlung auch Ladungsträger aufweisen, z.B. gestreute Elektronen. Störstrahlung ist insbesondere im Bereich des Ladungsträgeraustrittsfensters nachteilig, da üblicherweise mindestens der Kopf der Behandlungseinrichtung, an dem das Ladungsträgeraustrittsfenster angeordnet ist durch eine Öffnung in das Behältnis eingeführt wird. Die Öffnung des Behältnisses ist üblicherweise eine der schmalsten Stellen des Behältnisses, so dass die Behandlungseinrichtung und auch das Ladungsträgeraustrittsfenster der Wandung des Behältnisses sehr nahe kommen. Störstrahlung kann daher die Wandung in diesem Bereich mit sehr hoher Energie erreichen und wie oben dargestellt zu einer unzureichenden Sterilisierung dieses Bereichs oder sogar einer Schädigung des Behältnismaterials führen.

Der außerhalb der Behandlungseinrichtung entstehende Anteil der Störstrahlung verteilt sich über einen Bereich, dessen Größe auch von der Größe der Ladungsträgerwolke abhängt. Bei einer größeren Ladungsträgerwolke ist auch der Bereich, in welchem Störstrahlung abgegeben wird, größer als bei einer kleineren Ladungsträgerwolke. Daher kommt es bei einer festen Größe, insbesondere bei einer festen Länge des Vorsprungs und bei einer großen Ladungsträgerwolke verstärkt vor, dass Störstrahlung nicht auf den Vorsprung trifft, sondern diesen ungehindert passieren kann.

Um diesen Effekt zu reduzieren, ist in einer bevorzugten Ausführungsform der Vorrichtung zum Sterilisieren von Behältnissen das in den Bereich der aus dem Ladungsträgeraustrittsfenster austretenden Ladungsträger abgebbare Medium dazu geeignet, die Dimension einer durch die ausgetretenen Ladungsträger gebildete Ladungsträgerwolke derart zu verkleinern, dass Störstrahlung zu mindestens 25%, bevorzugt mindestens 50%, bevorzugt mindestens 60%, bevorzugt mindestens 70%, bevorzugt mindestens 80%, besonders bevorzugt mindestens 90% auf den gegenüber dem Ladungsträgeraustrittsfenster vorstehenden Vorsprung leitbar und von diesem absorbierbar ist.

Es wäre jedoch auch eine Vergrößerung der Ladungsträgerwolke denkbar.

Dadurch ist es möglich, die Dimension der Ladungsträgerwolke zumindest temporär so weit zu reduzieren, dass zumindest der außerhalb der Behandlungseinrichtung entstehende Anteil der Störstrahlung in größeren Anteilen auf den Vorsprung treffen und von diesem absorbiert werden kann.

Die Möglichkeit zur Variation der Geometrie einer Elektronenwolke kann auch einer Verbesserung der Sicherheitstechnik dienen. Bei Vorliegen bestimmter kritischer Verfahrensparameter kann in Abhängigkeit der Detektions- und der Reaktionsgeschwindigkeit der beschriebenen Vorrichtung das Einleiten von geeignetem Medium, z.B. Gas zum Erzeugen einer größeren Dichte, zur schlagartigen Verkleinerung (Verringerung der Dimension) der Elektronenwolke genutzt werden. Die Erzeugung von Röntgenstrahlung wird dadurch zwar nur minimal beeinflusst, jedoch wird der Erzeugungsort (für Störstrahlung, insbesondere für Röntgenstrahlung) näher an den Strahler bzw. die Behandlungseinrichtung verschoben, im Idealfall sehr nahe an das eigentliche Austrittsfenster. Der Entstehungsort von Störstrahlung wird somit in die Nähe des metallischen Fingers gelegt, so dass bei entsprechender Ausgestaltung des Fingers eine erste Umlenkung erreicht wird. In der Folge davon hat die weiter vorhandene Abschirmung weniger Störstrahlung abzuschirmen. Ein Anteil der Störstrahlung kann bereits nahe am Entstehungsort durch den Vorsprung absorbiert, umgewandelt, umgelenkt oder auf andere Weise unschädlich gemacht werden.

Bevorzugt ist eine Ausführungsform der Vorrichtung zum Sterilisieren von Behältnissen bei welcher durch die Medienleitung ein Medium führbar ist, welches zumindest eine physikalische Eigenschaft, bevorzugt die Dichte, eines weiteren im Bereich der durch die ausgetretenen Ladungsträger gebildeten Ladungsträgerwolke angeordneten Mediums, bevorzugt eines Gases, zu verändern. Wie bereits erwähnt, beschränkt sich die Auswahl der Medien nicht nur auf Medien, welche die Dichte des im Bereich der durch die ausgetretenen Ladungsträger gebildeten Ladungsträgerwolke angeordneten Mediums verändern, sondern schließt auch Medien ein, die andere physikalische Eigenschaften, bevorzugt dessen Wirkungsquerschnitt, dessen Stoßquerschnitt und/oder dessen Wechselwirkung mit Ladungsträgern verändern.

Besonders bevorzugt ist eine Ausführungsform der Vorrichtung zum Sterilisieren von Behältnissen bei der die Medienleitung mindestens abschnittsweise eine gekrümmte Form aufweist, wobei die Medienleitung bevorzugt derart gestaltet ist, dass das darin führbare Medium durch die Medienaustrittsöffnung in Richtung der Ladungsträgerwolke (d.h. mit einer (Vektor-) Komponente in diese Richtung), besonders bevorzugt im Wesentlichen entgegen einer Beschleunigungsrichtung der Ladungsträger, abgebbar ist. Vorzugsweise ist hier wenigstens ein Abschnitt der Medienleitung gekrümmt und bevorzugt um einen Winkel gekrümmt, der größer ist als 60°, bevorzugt größer als 90°, bevorzugt größer als 120°, bevorzugt größer als 150°.

Es könnten jedoch auch Ablenkeinrichtungen zum Ablenken des Mediums vorgesehen sein, welche z.B. an der Medienaustrittsöffnung angeordnet sein könnten.

Durch diese Ausführungsform strömt das Medium den beschleunigten Ladungsträgern im Wesentlichen entgegen. Dies hat einerseits den Vorteil, dass durch den Massestrom und daraus folgender Wechselwirkung mit dem Massestrom der Ladungsträger, diese bereits in geringem Maße abgelenkt, insbesondere abgebremst werden. Noch größeren Einfluss hat in diesem Zusammenhang jedoch, dass durch diese Ausführungsform gewährleistet werden kann, dass kontinuierlich Medium in Richtung des Ladungsträgeraustrittsfensters strömt und andere Medien in dem Bereich vor dem Austrittsfenster so kontinuierlich verdünnt oder sogar aus diesem Bereich nahezu vollständig verdrängt werden. Das in Richtung des Ladungsträgeraustrittsfensters strömende Medium sorgt bei dieser Ausführungsform dafür, dass die Konzentration des Mediums im Bereich des Ladungsträgeraustrittsfensters sehr hoch ist und Ladungsträger durch Wechselwirkungen mit dem Medium bereits unmittelbar nach dem Durchtritt des Ladungsträgeraustrittsfensters abgebremst werden. Die Bildung von Reservoiren im Bereich des Ladungsträgeraustrittsfensters, in denen das zuvor vorhandene Medium nicht durch das neue, aus der Medienaustrittsöffnung abgegebene Medium ersetzt wird, wird verhindert. Bei einer Abgabe des Mediums in einer im Wesentlichen parallel zur Austrittsrichtung der Ladungsträger aus der Behandlungseinrichtung verlaufenden Richtung wäre es vorstellbar, dass insbesondere in Bereichen, welche im Strömungsschatten des Vorsprungs liegen, Reste des zuvor vorhandenen Mediums verbleiben.

In einer besonders bevorzugten Ausführungsform der Vorrichtung zum Sterilisieren von Behältnissen verläuft der gegenüber dem Ladungsträgeraustrittsfenster vorstehende Vorsprung zumindest abschnittsweise entlang eines Umfangs der Behandlungseinrichtung, bevorzugt vollumfänglich, besonders bevorzugt in Form eines (insbesondere geraden) Kreiszylinders.

Der Vorsprung kann als einzelnes Element ausgebildet sein oder auch aus einer Vielzahl von Elementen bestehen. Der Vorsprung ist bevorzugt so angeordnet, dass er zwischen einem Entstehungsort für Störstrahlung und einem besonders vor dieser Strahlung zu schützenden Bereich liegt. Im Fall, dass beispielsweise ein enger Hals eines Behältnisses vor der Störstrahlung geschützt werden soll, ist es vorteilhaft, wenn auch der Vorsprung den Entstehungsort für Störstrahlung entlang eines vollen Umfangs umgibt. So kann der Behältnishals, welcher die Behandlungseinrichtung bzw. einen Kopf der Behandlungseinrichtung vollumfänglich umgibt gleichmäßig von Störstrahlung abgeschirmt werden.

Die erwähnte Ausführungsvariante des Vorsprungs in Form eines geraden Kreiszylinders ist bevorzugt, da so bei geringem Materialbedarf ein besonders großer Bereich vor Störstrahlung geschützt werden kann. Es sind jedoch wie bereits erwähnt auch Ausführungen des Vorsprungs möglich, in welchen der Vorsprung um einen Winkel bis zu ± 10°, ± 20°, ± 30°oder sogar bis ± 45° von der Senkrechten abweicht. Dabei sind insbesondere Winkel bevorzugt bei welchen das vom Ladungsträgeraustrittsfenster entfernte Ende des Vorsprungs in Richtung der durch das Zentrum des Ladungsträgeraustrittsfensters verlaufenden Längsachse der Behandlungseinrichtung geneigt ist. Bei einer vollumfänglichen Ausführung des Vorsprungs weist dieser somit die Form eines Kegelstumpfes auf. Dadurch wird ermöglicht, dass auf den Vorsprung treffende Störstrahlung bevorzugt in Richtung des Ladungsträgeraustrittsfensters reflektiert wird, wo sie durch andere in die Behandlungseinrichtung integrierte Abschirmeinrichtungen vom unkontrollierten Übergang in die Umgebung abgehalten werden kann. Eine unkontrollierte Abstrahlung kann somit stark reduziert werden.

Es ist auch möglich, den Vorsprung in einer mehrlagigen Variante auszuführen. In dieser Variante ist eine der Ladungsträgerwolke zugewandte Seite des Vorsprungs im Wesentlichen aus einem Abschirmmaterial bestehen, welches im Wesentlichen Atome niedriger Ordnungszahl aufweist. Atome niedriger Ordnungszahl haben bei der Absorption einiger Ladungsträgerarten, z.B. Elektronen, den Vorteil, dass nur ein geringer Anteil der kinetischen Energie der Ladungsträger in Bremsstrahlung, insbesondere in Röntgenstrahlung, umgewandelt wird. Bevorzugt handelt es sich um eine Metallschicht, bevorzugt aus einem Leichtmetall, z.B. Aluminium. Es sind jedoch auch nichtmetallische Materialien wie z.B. Kunststoffe denkbar.

Radial außerhalb dieser Abschirmmaterialschicht aus Atomen niedriger Ordnungszahl ist innerhalb des Vorsprungs eine weitere Schicht angeordnet, die insbesondere zur Abschirmung der Umgebung gegenüber Röntgenstrahlung geeignet ist. Diese Schicht besteht im Wesentlichen aus mindestens einem Metall großer Ordnungszahl (Schwermetall), da diese Röntgenstrahlung besonders gut absorbieren. Die Umgebung kann so auch vor der als Bremsstrahlung in der der Ladungsträgerwolke zugewandten Schicht des Vorsprungs entstehenden Röntgenstrahlung geschützt werden.

In einer bevorzugten Ausführungsform der Vorrichtung zum Sterilisieren von Behältnissen ist die Medienleitung zumindest abschnittsweise mit der Behandlungseinrichtung verbunden, bevorzugt in diese integriert und gemeinsam mit der Behandlungseinrichtung bewegbar. Ein großer Vorteil dieser Ausführungsform ist, dass eine gemeinsame Einrichtung zum Einführen der Behandlungseinrichtung und der Medienleitung in das Behältnis verwendet werden kann. Dies stellt jedoch besonders Anforderungen an die Dimensionierung der Behandlungseinrichtung, da diese einen besonders geringen Querschnitt aufweisen muss.

Dabei ist es möglich, dass die Medienleitung seitlich an der Behandlungseinrichtung angeordnet ist. In diesem Fall verläuft die Medienleitung bevorzugt radial außerhalb des Vorsprungs.

Es ist auch möglich, dass mehrere Medienleitungen seitlich an der Behandlungseinrichtung angeordnet sind. Dies ist besonders vorteilhaft, wenn eine sehr homogene Abgabe des Mediums gefordert wird. Beispielsweise kann dies notwendig sein, wenn eine symmetrische Form der Ladungsträgerwolke gefordert ist und die Ladungsträger durch einseitige Aufgabe des Mediums auf die der Medienaustrittsöffnung in Bezug auf eine Längsache der Behandlungseinrichtung entgegengesetzte Seite der Behandlungseinrichtung gedrückt würden.

Weiterhin ist es in einer Ausführungsform vorgesehen, dass die Medienleitung zumindest abschnittsweise in die Behandlungseinrichtung integriert ist. Bei dieser Ausführungsform verläuft die Medienleitung bevorzugt radial innerhalb des Vorsprungs. Diese Ausführungsform ermöglicht eine besonders einfache Zuführung des Mediums in den radial innerhalb eines vollumfänglich entlang eines Umfangs der Behandlungseinrichtung verlaufenden Vorsprungs befindlichen Bereich, d.h. den Bereich, welcher direkt in Abstrahlrichtung der Ladungsträger vor dem Ladungsträgeraustrittsfenster angeordnet ist. Dadurch kann besonders effektiv einem Verbleib von Resten des zuvor in diesem Bereich befindlichen Mediums vorgebeugt werden. Bevorzugt befindet sich eine Medienleitung und/oder eine Medienaustrittsöffnung in einem Bereich, welcher zwischen Vorsprung und Ladungsträgeraustrittsfenster angeordnet ist. Bei einem ringförmigen Ladungsträgeraustrittsfenster ist es möglich, dass eine Medienleitung und/oder eine Medienaustrittsöffnung im Zentrum des Ladungsträgeraustrittsfensters angeordnet ist.

Bevorzugt verläuft die Medienleitung zumindest abschnittsweise parallel zu einer Außenwandung der Behandlungseinrichtung. Besonders bevorzugt verläuft die Medienleitung zumindest abschnittsweise parallel zu Längsachse des Vorsprungs.

Bevorzugt weist die Behandlungseinrichtung ein äußeres Gehäuse auf, welches das Ladungsträgeraustrittsfenster aufweist. Zur Kühlung des Ladungsträgeraustrittsfenster ist bevorzugt vorgesehen, dass innerhalb des äußeren Gehäuses und von diesem beabstandet ein inneres Gehäuse angeordnet ist, so dass zwischen dem inneren Gehäuse und dem äußeren Gehäuse ein Bereich entsteht, in welchem ein Kühlmedium führbar ist. Bevorzugt kann dieser Bereich in verschiedene Abteilungen unterteilt sein, in welchen verschiedene Medien geführt werden können und/oder in denen das Medium in verschiedene Richtungen strömt. So kann beispielsweise ein definierter Zu- und Abfluss des Kühlmediums zu dem Ladungsträgeraustrittsfenster und von diesem weg eingestellt werden.

Weiterhin ist eine Anlage zum Behandeln von Behältnissen Gegenstand der vorliegenden Erfindung, welche mindestens eine Vorrichtung, bevorzugt mehrere Vorrichtungen zum Sterilisieren von Behältnissen und insbesondere einer Innenwandung von Behältnissen mittels beschleunigter Ladungsträger mit einer Ladungsträgerquelle zum Erzeugen der Ladungsträger, mit einer Beschleunigungseinrichtung, durch welche Ladungsträger in Richtung eines Ladungsträgeraustrittsfensters beschleunigbar sind, wobei das Ladungsträgeraustrittsfenster an einer Behandlungseinrichtung angeordnet ist, welche entlang einer Einführrichtung durch eine Öffnung in das Behältnis einführbar ist, um eine Innenwandung des Behältnisses mit den aus dem Ladungsträgeraustrittsfenster austretenden Ladungsträgern zu beaufschlagen, wobei die Behandlungseinrichtung eine Medienleitung mit einer Medienaustrittsöffnung aufweist, deren Medienaustrittsöffnung bevorzugt durch die Öffnung in das Behältnis einführbar ist, wobei die Behandlungseinrichtung mindestens einen in Einführrichtung gegenüber dem Ladungsträgeraustrittsfenster vorstehenden Vorsprung aufweist, welcher dazu geeignet ist, Störstrahlung zu absorbieren und wobei durch die Medienleitung ein Medium führbar ist, welches durch die Medienaustrittsöffnung zumindest in den Bereich der aus der Ladungsträgeraustrittsfenster austretenden Ladungsträger abgebbar ist, wobei das Medium dazu geeignet ist, die Dimension einer durch die ausgetretenen Ladungsträger gebildeten Ladungsträgerwolke zu verändern.

Durch eine derartige Anlage ist es möglich, eine Sterilisierung von Behältnissen insbesondere in den zur Produktion und Befüllung von Behältnissen verwendeten hohen Taktraten und Durchsatzzahlen durchzuführen.

Bevorzugt weist eine derartige Anlage eine weitere Vorrichtung zum Sterilisieren auf, bevorzugt zum Sterilisieren einer Außenwandung der Behältnisse.

In einer weiteren bevorzugten Ausführungsform weist die Anlage eine Einführeinrichtung auf, mittels welcher die Behandlungseinrichtung in das Innere eines Behältnisses einführbar ist, wobei das Behältnis und die Behandlungseinrichtung im Bereich der Einführeinrichtung gegenüber einander (insbesondere in einer Längsrichtung des Behältnisses) relativbeweglich sind, wobei die Relativbeweglichkeit durch Bewegung der Behandlungseinrichtung in Richtung des Behältnisses, durch Bewegung des Behältnisses in Richtung der Behandlungseinrichtung und/oder einer Kombination beider Bewegungen realisierbar ist.

Je nach Ausführungsform der Anlage kann es vorteilhaft sein, die Behandlungseinrichtung in Richtung des Behältnisses zu bewegen, um sie zu dessen Sterilisierung in dieses einzuführen. Bei besonders aufwändiger Vorrichtung zur Erzeugung und Beschleunigung der Ladungsträger ist die genaue Steuerung der Behandlungseinrichtung jedoch evtl. sehr aufwändig, so dass eine Bewegung des Behältnisses in Richtung der Behandlungseinrichtung vorteilhaft ist. In einigen Ausführungsformen kann auch eine Kombination beider oben genannter Bewegungen vorteilhaft sein, beispielsweise um ein besonders schnelles Einführen der Behandlungseinrichtung in das Behältnis zu ermöglichen.

Bevorzugt weist die Anlage eine Transporteinrichtung auf, welche die Behältnisse entlang eines vorgegebenen Transportpfades insbesondere auch während deren Sterilisation bewegt. Vorteilhaft handelt es sich bei der Transporteinrichtung um einen drehbaren Träger, an dem besonders bevorzugt eine Vielzahl von Greifelementen angeordnet ist.

Vorzugsweise weist die Anlage eine Einrichtung zum Befüllen von Behältnissen auf und die erfindungsgemäße Vorrichtung ist stromaufwärts bezüglich dieser Einrichtung angeordnet.

Bevorzugt weist die Anlage eine weitere Vorrichtung zum Sterilisieren einer Außenwandung der Behältnisse auf

Weiterhin ist bevorzugt, dass die Anlage mindestens ein Transportelement, bevorzugt einen Transportstern aufweist, welcher dazu geeignet ist, ein Behältnis von einer Vorrichtung zum Sterilisieren von Behältnissen zu übernehmen und an eine weitere Vorrichtung zum Sterilisieren zu übergeben.

Ein weiterer wesentlicher Aspekt der Erfindung ist ein Verfahren zum Sterilisieren von Behältnissen nach Anspruch 6. Durch dieses Verfahren ist es somit möglich, Einflussnahme auf die Geometrie der Elektronenwolke zu nehmen und deren Dimension zu verändern. Hierfür werden Medien, z.B. Fluide, insbesondere Gase und hier bevorzugt schwerere oder leichtere Substanzen als Luft, in die Bestrahlungskammer, insbesondere in den Bereich der Ladungsträgerwolke eingeleitet. Alternativ oder ergänzend kann auch durch Luftzu- oder -abfuhr der Druck in der Kammer, bevorzugt im Bereich der Ladungsträgerwolke erhöht oder verringert werden. Durch diese Maßnahmen wird die Dichte in der Prozesskammer und insbesondere im Bereich der Ladungsträgerwolke erhöht oder erniedrigt und somit eine beinahe sofortige Reduzierung der Ausmaße der Ladungsträgerwolke, z.B. einer Elektronenwolke, ermöglicht. Die Möglichkeit der Druckvariation erlaubt es, durch gezieltes Einblasen von Luft oder einem anderen Medium in das Behältnis den Sterilisationsprozess schlagartig zu unterbrechen. Hierdurch ergeben sich besondere Möglichkeiten zur Ergänzung der (bestehenden) Sicherheitstechnik.

Bevorzugt ist eine Variante des Verfahrens zum Sterilisieren von Behältnissen und insbesondere einer Innenwandung von Behältnissen mittels beschleunigter Ladungsträger bei dem das in den Bereich der aus dem Ladungsträgeraustrittsfenster austretenden Ladungsträger abgegebene Medium die Dimension einer durch die ausgetretenen Ladungsträger gebildete Ladungsträgerwolke derart verkleinert, dass Störstrahlung zu mindestens 25%, bevorzugt mindestens 50%, bevorzugt mindestens 60%, bevorzugt mindestens 70%, bevorzugt mindestens 80%, besonders bevorzugt mindestens 90% auf den gegenüber dem Ladungsträgeraustrittsfenster vorstehenden Vorsprung geleitet wird und von diesem absorbierbar ist.

Diese Verfahrensvariante ermöglicht es, eine zur Sterilisierung eines großen Behältnisses benötigte Elektronenwolke so weit zu verkleinern, dass auch ein wesentlich kleineres Behältnis mit der für dieses Behältnis optimalen Strahlungsintensität an dessen Innenoberfläche sterilisiert werden kann. Insbesondere bei einem Wechsel der Sterilisierung zwischen Preforms und Getränkeflaschen ist es notwendig, die Dimension der Ladungsträgerwolke um einen großen Faktor zu verändern.

Weiterhin bevorzugt ist eine Variante des Verfahrens zum Sterilisieren von Behältnissen und insbesondere einer Innenwandung von Behältnissen mittels beschleunigter Ladungsträger wobei bei einer Reduzierung der Dimension der durch die ausgetretenen Ladungsträger gebildete Ladungsträgerwolke durch das Medium die Intensität der Störstrahlung durch den gegenüber dem Ladungsträgeraustrittsfenster vorstehenden Vorsprung auf ein Maß reduziert wird, bei dem bei einem Wechsel des zu sterilisierenden Behältnisses eine Unterbrechung der Beschleunigung der Ladungsträger aus der Ladungsträgerquelle mit der Beschleunigungseinrichtung in Richtung des Ladungsträgeraustrittsfensters überflüssig wird.

Durch diese Verfahrensvariante ist es möglich, die Ladungsträgerwolke so weit zu verkleinern, dass Störstrahlung zu einem hohen Anteil in dem Bereich auftritt, welcher vor dem Ladungsträgeraustrittsfenster und neben dem gegenüber dem Ladungsträgeraustrittsfenster vorstehenden Vorsprung angeordnet ist. In diesem Bereich ist es besonders gut möglich, Störstrahlung durch den Vorsprung zu absorbieren oder in andere Strahlungsarten umzuwandeln. Dementsprechend kann daher die von der Behandlungseinrichtung unkontrolliert abgegebene Strahlung auch außerhalb eines Behältnisses sehr weit reduziert werden. Dadurch ist es möglich, die Behandlungseinrichtung aus dem Behältnis zu entfernen ohne die Erzeugung von Ladungsträgern oder deren Beschleunigung mittels der Beschleunigungseinrichtung abzuschalten oder zu reduzieren. Dadurch kann auf entsprechende Steuereinrichtungen verzichtet werden und die Behandlungseinrichtung besonders einfach und kompakt ausgeführt werden. Dies kann Vorteile bei den Herstell- und Anschaffungskosten bringen und vereinfacht die Möglichkeit der Bewegung der Behandlungseinrichtung.

Weitere Vorteile und Ausführungsformen ergeben sich aus den beigefügten Zeichnungen:
Darin zeigen:

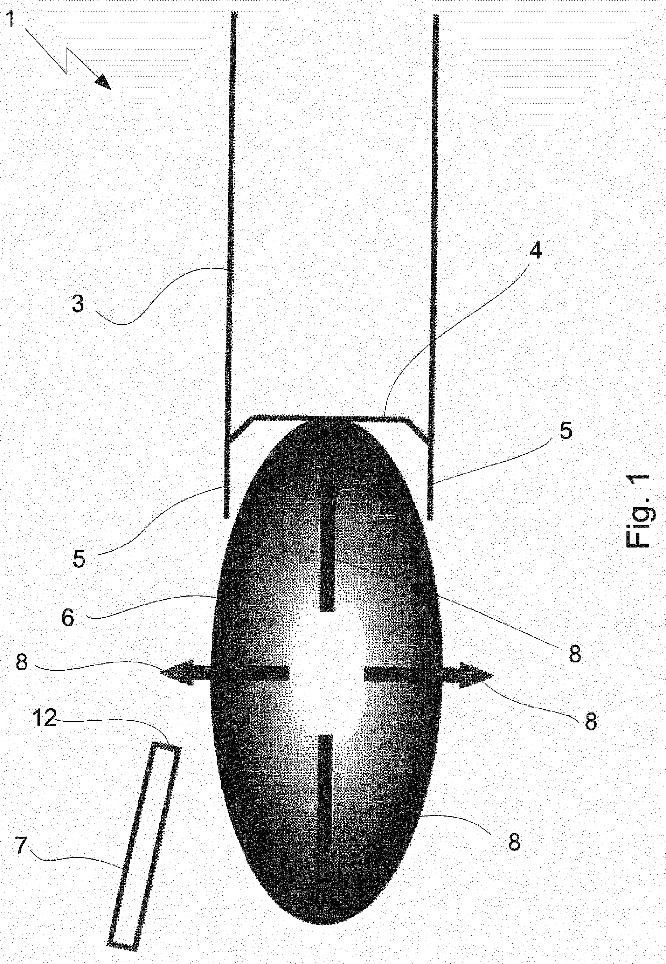

   - Fig. 1: eine schematische Darstellung einer Seitenansicht auf eine Behandlungseinrichtung mit gegenüber dem Ladungsträgeraustrittsfenster vorstehenden Vorsprung zum Sterilisieren von Behältnissen mittels beschleunigter Ladungsträger ohne Zuführung des Mediums zur Veränderung der Dimension einer durch die ausgetretenen Ladungsträger gebildeten Ladungsträgerwolke;

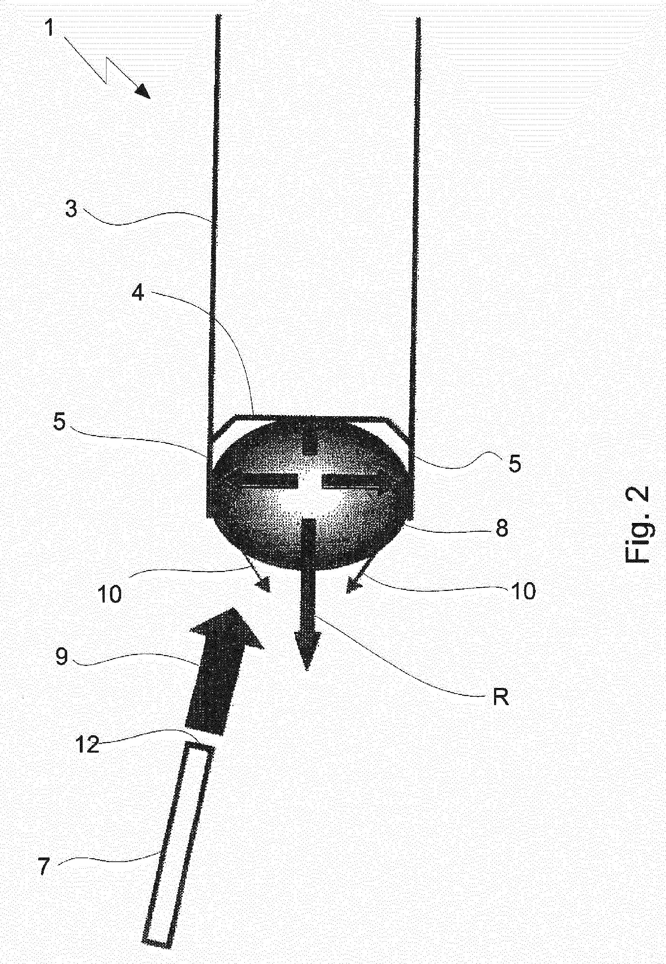

   - Fig. 2: eine schematische Darstellung einer Seitenansicht auf eine Behandlungseinrichtung mit gegenüber dem Ladungsträgeraustrittsfenster vorstehenden Vorsprung zum Sterilisieren von Behältnissen mittels beschleunigter Ladungsträger während der Zuführung des Mediums zur Veränderung der Dimension einer durch die ausgetretenen Ladungsträger gebildeten Ladungsträgerwolke;

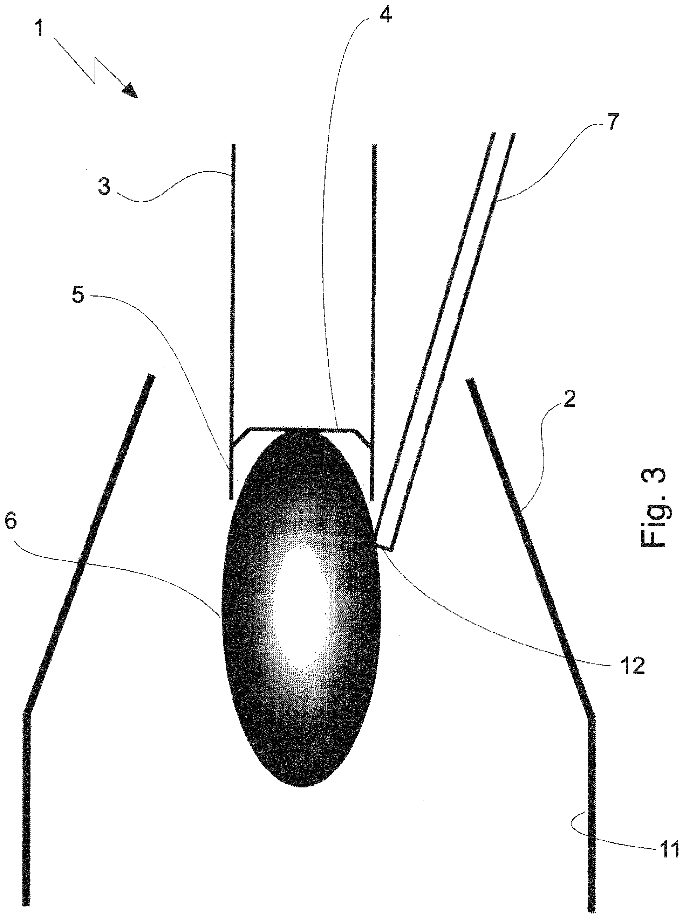

   - Fig. 3: eine schematische Darstellung einer Seitenansicht auf eine Behandlungseinrichtung mit gegenüber dem Ladungsträgeraustrittsfenster vorstehenden Vorsprung zum Sterilisieren von Behältnissen mittels beschleunigter Ladungsträger, welche in ein Behältnis eingeführt ist.

Figur 1 zeigt eine schematische Darstellung einer Seitenansicht auf eine Behandlungseinrichtung 1. Diese weist eine gegenüber dem Ladungsträgeraustrittsfenster 4 vorstehenden Vorsprung 5 auf. Die Vorrichtung zum Sterilisieren von Behältnissen 2 mittels beschleunigter Ladungsträger ist hierbei ohne Zuführung des Mediums 9 zur Veränderung der Dimension einer durch die ausgetretenen Ladungsträger gebildeten Ladungsträgerwolke 6 dargestellt.

Von der Behandlungseinrichtung 1 ist ein zumindest abschnittsweise in ein Behältnis 2 einführbarer Strahlfinger 3 gezeigt, aus dem die Ladungsträger austreten. Die Ladungsträgerquelle zum Erzeugen der Ladungsträger und die Beschleunigungseinrichtung, durch welche Ladungsträger in Richtung R eines Ladungsträgeraustrittsfensters 4 beschleunigbar sind, sind nicht gezeigt. Das Ladungsträgeraustrittsfenster 4 befindet sich in einer Wandung des Strahlfinger 3, welche in ein Behältnis 2 einführbar ist. Das Ladungsträgeraustrittsfenster 4 befindet sich in einem Strahlengang der Ladungsträger und ermöglicht das Hindurchtreten der Ladungsträger aus dem Inneren des Strahlfingers 3 in die Umgebung.

Außerdem ist eine Medienleitung 7 mit einer Medienaustrittsöffnung 12 gezeigt.

In Abhängigkeit der erzeugten Ladungsträger und der mittels der Beschleunigungseinrichtung auf die Ladungsträger übertragenen kinetischen Energie entsteht auf der Außenseite des Ladungsträgeraustrittsfensters 4 eine Ladungsträgerwolke 6 einer definierten Ausdehnung, welche ein Behältnis 2 sterilisieren kann.

Seitlich des Ladungsträgeraustrittsfensters 4 ist ein Vorsprung 5 angeordnet, welcher sich in Ladungsträgerstrahlrichtung über das Gehäuse 3 des Strahlfingers hinaus erstreckt. Im gezeigten Beispiel handelt es sich um einen umlaufenden Vorsprung 5 in Form eines geraden Kreiszylinders, dessen äußerer Radius demjenigen des Strahlfingers 3 entspricht. Dies hat den Vorteil, dass am Übergang zwischen dem Gehäuse des Strahlfingers 3 und dem Vorsprung 5 keine Unebenheiten auf der Außenseite entstehen und so ein Hängenbleiben beim Ein- oder Ausführen in ein bzw. aus einem Behältnis 2 vermieden werden kann.

Der Vorsprung 5 weist im gezeigten Beispiel eine Länge auf, die etwa dem Radius des Gehäuses des Strahlfingers 3 entspricht. Es sind jedoch auch deutlich davon abweichende Längen des Vorsprungs 5 möglich. Bevorzugt beträgt die Länge des Vorsprungs 5 mindestens 1/10, bevorzugt 1/5, besonders bevorzugt 1/3 des Radius des Strahlfingergehäuses. Eine maximale Länge des Vorsprungs 5 ist nicht definiert, jedoch haben sich Längen von weniger als einer halben Länge des zu sterilisierenden Behältnisses 2, bevorzugt weniger als der 10-fachen Länge des Radius des Strahlfingergehäuses, bevorzugt weniger als der 5-fachen Länge des Radius des Strahlfingergehäuses, besonders bevorzugt weniger als der 3-fachen Länge des Radius des Strahlfingergehäuses als vorteilhaft erwiesen.

Der Vorsprung 5 kann somit Störstrahlung 8, in diesem Fall insbesondere Streustrahlung, welche durch Reflexionen und Streuung im Inneren des Strahlfingers 3 und durch Wechselwirkung mit dem Ladungsträgeraustrittsfenster 4 entsteht, absorbieren oder abschirmen.

Störstrahlung 8, die außerhalb des durch den Vorsprung 5 begrenzten Bereichs aus der Ladungsträgerwolke 6 entsteht, ist durch die Pfeile 8 schematisch symbolisiert. Je nach Richtung der Störstrahlung 8 kann diese auf die Behandlungseinrichtung 1 einstrahlen oder auch in die Umgebung abgestrahlt werden.

In Figur 2 ist eine schematische Darstellung einer Seitenansicht auf eine Behandlungseinrichtung 1 mit gegenüber dem Ladungsträgeraustrittsfenster 4 vorstehenden Vorsprung 5 zum Sterilisieren von Behältnissen 2 mittels beschleunigter Ladungsträger während der Zuführung des Mediums 9 zur Veränderung der Dimension einer durch die ausgetretenen Ladungsträger gebildeten Ladungsträgerwolke 6 gezeigt.

Im Wesentlichen unterscheidet sich die in Fig. 2 dargestellte Situation dadurch von der in Fig.1 dargestellten, dass aus der Medienleitung 7 mit einer Medienaustrittsöffnung 12 ein Medium 9 in den Bereich außerhalb der Behandlungseinrichtung 1 geleitet wird. Dieses Medium 9 bewirkt eine Veränderung der Reichweite der durch das Ladungsträgeraustrittsfenster 4 austretenden Ladungsträger und verringert so in dem dargestellten Fall die Größe der Ladungsträgerwolke 6.

Daraus resultiert, dass auch die außerhalb des Strahlfingers 3 gebildete Ladungsträgerwolke 6 deutlich verkleinert wird und, daraus entstehende, durch die Pfeile 8 schematisch symbolisierte Störstrahlung 8 in einem größeren Anteil in einem Bereich abgestrahlt wird, aus welchem sie auf den Vorsprung 5 trifft. Beim Auftreffen auf den Vorsprung 5 kann diese Strahlung absorbiert oder umgewandelt werden, so dass der Anteil an unkontrolliert in die Umgebung abgegebener Strahlung deutlich reduziert ist.

Die dünneren Pfeile 10 symbolisieren Reststrahlung 10, welche von dem Vorsprung 5 weder absorbiert noch umgewandelt sondern reflektiert wurde. Deren Anteil lässt sich je nach Ladungsträgerart, der im Vorsprung 5 verwendeten Materialien und der Geometrie des Vorsprungs 5 den Anforderungen entsprechend reduzieren.

Fig. 3 zeigt eine schematische Darstellung einer Seitenansicht auf eine Behandlungseinrichtung 1 mit gegenüber dem Ladungsträgeraustrittsfenster 4 vorstehenden Vorsprung 5 zum Sterilisieren von Behältnissen 2 mittels beschleunigter Ladungsträger, welche in ein Behältnis 2 eingeführt ist.

Von der Behandlungseinrichtung 1 ist auch in dieser Fig. lediglich der in das Behältnis 2 einführbare Strahlfinger 3 mit dem Vorsprung 5 gezeigt. Ebenso ist die Ladungsträgerwolke 6 zu erkennen, welche durch das Ladungsträgeraustrittsfenster 4 aus dem Strahlfinger 3 in das Behältnis 2 austritt. Die Ladungsträgerquelle zum Erzeugen der Ladungsträger und die Beschleunigungseinrichtung, durch welche Ladungsträger in Richtung eines Austrittfensters beschleunigbar sind, sind nicht gezeigt. Seitlich des Strahlfingers 3 ist die Medienleitung 7 mit einer Medienaustrittsöffnung 12 gezeigt, welche ebenfalls zumindest abschnittsweise in das Behältnis 2 eingeführt ist.

Die Medienaustrittsöffnung 12 befindet sich in der Nähe der Ladungsträgerwolke 6, so dass austretendes Medium 9 diese sofort beeinflussen kann.

Im Gegensatz zu den in den Fig. 1 und 2 gezeigten Ausführungsformen verläuft in dem in Fig. 3 gezeigten Beispiel die Medienleitung 7 bis zu der Medienaustrittsöffnung 12 im Wesentlichen Parallel zu der Ladungsträgerstrahlrichtung. Diese Ausführungsform ist weniger bevorzugt, da so in dem Bereich, welcher sich durch den Vorsprung 5 geschützt, unmittelbar vor dem Ladungsträgeraustrittsfenster 4 befindet, der Austausch des Mediums 9 verzögert ist.

Soll jedoch die Änderung der Dimension der Ladungsträgerwolke 6 der Anpassung an die Behältnisgröße dienen, ist die gezeigte Ausführungsform vorteilhaft, da so bei geringem vorrichtungsseitigem Aufwand ein schneller Medienaustausch im Behältnis 2 erfolgen kann. Die gerade Ausführung der Medienleitung 7 erlaubt besonders schnelle Medienströme.

### Bezugszeichenliste

- 1: Vorrichtung zum Sterilisieren von Behältnissen, Behandlungseinrichtung
- 2: Behältnis
- 3: Strahlfinger, Gehäuse
- 4: Ladungsträgeraustrittsfenster
- 5: Vorsprung
- 6: Ladungsträgerwolke
- 7: Medienleitung
- 8: Pfeil, Störstrahlung
- 9: Medium
- 10: Pfeil, Reststrahlung
- 11: Innenwandung
- 12: Medienaustrittsöffnung
- R: Ladungsträgerstrahlrichtung

## Patentansprüche

1. Vorrichtung (1) zum Sterilisieren einer Innenwandung von Behältnissen (2) mittels beschleunigter Ladungsträger mit einer Ladungsträgerquelle zum Erzeugen der Ladungsträger, mit einer Beschleunigungseinrichtung, durch welche Ladungsträger in Richtung eines Ladungsträgeraustrittsfensters (4) beschleunigbar sind, wobei das Ladungsträgeraustrittsfenster (4) an einer Behandlungseinrichtung (1) angeordnet ist, weiche entlang einer Einführrichtung durch eine Öffnung in das Behältnis (2) einführbar ist, um eine Innenwandung des Behältnisses (2) mit den aus dem Ladungsträgeraustrittsfenster (4) austretenden Ladungsträgern zu beaufschlagen, wobei die Behandlungseinrichtung (1) eine Medienleitung (7) mit einer Medienaustrittsöffnung (12) aufweist, deren Medienaustrittsöffnung (12) durch die Öffnung in das Behältnis (2) einführbar ist,
**dadurch gekennzeichnet, dass**
die Behandlungseinrichtung (1) mindestens einen in Einführrichtung gegenüber dem Ladungsträgeraustrittsfenster (4) vorstehenden Vorsprung (5) aufweist, welcher dazu geeignet ist, Störstrahlung (8) zu absorbieren und wobei durch die Medienleitung (7) ein Medium (9) führbar ist, welches durch die Medienaustrittsöffnung (12) zumindest in den Bereich der aus dem Ladungsträgeraustrittsfenster (4) austretenden Ladungsträger abgebbar ist, wobei das Medium (9) dazu geeignet ist, die Dimension einer durch die ausgetretenen Ladungsträger gebildeten Ladungsträgerwolke (6) zu verändern, wobei
- der gegenüber dem Ladungsträgeraustrittsfenster (4) vorstehende Vorsprung (5) zumindest abschnittsweise entlang eines Umfangs der Behandlungseinrichtung (1) verläuft,
- sich der Vorsprung (5) über das Ladungsträgeraustrittsfenster (4) hinaus erstreckt, in der Nähe des Ladungsträgeraustrittsfensters (4) angeordnet ist und der Vorsprung (5) sich von einer durch das Ladungsträgeraustrittsfenster (4) gebildeten Ebene mindestens auch in Richtung des austretenden Ladungsträgerstrahls erstreckt.

2. Vorrichtung (1) zum Sterilisieren einer Innenwandung von Behältnissen (2) nach Anspruch 1,
**dadurch gekennzeichnet, dass**,
die Medienleitung (7) mindestens abschnittsweise eine gekrümmte Form aufweist,
wobei die Medienleitung (7) bevorzugt derart gekrümmt ist, dass das darin führbare Medium (9) durch die Medienaustrittsöffnung (12) in Richtung der Ladungsträgerwolke (6), bevorzugt mit einer Komponente entgegen, besonders bevorzugt entgegen einer Beschleunigungsrichtung (R) der Ladungsträger, abgebbar ist.

3. Vorrichtung (1) zum Sterilisieren einer Innenwandung von Behältnissen (2) nach wenigstens einem der vorangegangenen Ansprüche,
**dadurch gekennzeichnet, dass**,
die Medienleitung (7) zumindest abschnittsweise mit der Behandlungseinrichtung (1) verbunden ist, bevorzugt in diese integriert ist und gemeinsam mit der Behandlungseinrichtung (1) bewegbar ist.

4. Anlage zum Behandeln einer Innenwandung von Behältnissen (2),
**dadurch gekennzeichnet, dass**
die Anlage eine Vielzahl von Vorrichtungen (1) zum Sterilisieren von Behältnissen (2) nach wenigstens einem der vorangegangenen Ansprüche aufweist.

5. Anlage zum Behandeln einer Innenwandung von Behältnissen (2) nach Anspruch 4,
**dadurch gekennzeichnet, dass**
die Anlage eine Einführeinrichtung aufweist, mittels welcher die Behandlungseinrichtung (1) in das Innere eines Behältnisses (2) einführbar ist, wobei das Behältnis (2) und die Behandlungseinrichtung (1) bevorzugt in einer Längsrichtung des Behältnisses (2) gegenüber einander relativbeweglich sind.

6. Verfahren zum Sterilisieren einer Innenwandung von Behältnissen (2) mittels beschleunigter Ladungsträger, wobei Ladungsträger in einer Ladungsträgerquelle erzeugt werden und mit einer Beschleunigungseinrichtung in Richtung eines Ladungsträgeraustrittsfensters (4) beschleunigt werden, wobei das Ladungsträgeraustrittsfenster (4) an einer Behandlungseinrichtung (1) angeordnet ist, welche entlang einer Einführrichtung durch die Öffnung in das Behältnis (2) eingeführt wird, um eine innenwandung des Behältnisses (2) mit den aus dem Ladungsträgeraustrittsfenster (4) austretenden Ladungsträgern zu beaufschlagen, wobei die Behandlungseinrichtung (1) eine Medienleitung (7) mit einer Medienaustrittsöffnung (12) aufweist, deren Medienaustrittsöffnung (12) durch die Öffnung in das Behältnis (2) einführbar ist,
**dadurch gekennzeichnet, dass**
die Behandlungseinrichtung (1) mindestens einen in Einführrichtung gegenüber dem Ladungsträgeraustrittsfenster (4) vorstehenden Vorsprung (5) aufweist, welcher Störstrahlung (8) absorbieren kann und wobei durch die Medienleitung (7) ein Medium (9) geführt werden kann, welches durch die Medienaustrittsöffnung (12) zumindest in den Bereich der aus dem Ladungsträgeraustrittsfenster (4) austretenden Ladungsträger abgebbar ist, wobei das Medium (9) die Dimension einer durch die ausgetretenen Ladungsträger gebildeten Ladungsträgerwolke (6) verändert, wobei
- der gegenüber dem Ladungsträgeraustrittsfenster (4) vorstehende Vorsprung (5) zumindest abschnittsweise entlang eines Umfangs der Behandlungseinrichtung (1) verläuft,
- sich der Vorsprung (5) über das Ladungsträgeraustrittsfenster (4) hinaus erstreckt, in der Nähe des Ladungsträgeraustrittsfensters (4) angeordnet ist und der Vorsprung (5) sich von einer durch das Ladungsträgeraustrittsfenster (4) gebildeten Ebene mindestens auch in Richtung des austretenden Ladungsträgerstrahls erstreckt.

## Claims

1. An apparatus (1) for sterilizing an inner wall of containers (2) by means of accelerated charge carriers with a charge carrier source for producing the charge carriers, with an acceleration device by which charge carriers are capable of being accelerated in the direction of a charge carrier emission window (4), wherein the charge carrier emission window (4) is arranged on a treatment device (1) which is capable of being introduced through an opening into the container (2) along an insertion direction, in order to act upon an inner wall of the container (2) with the charge carriers issuing from the charge carrier emission window (4), wherein the treatment device (1) has a medium line (7) with a medium emission opening (12), the medium emission opening (12) of which is preferably capable of being introduced through the opening into the container (2),
**characterized in that**
the treatment device (1) has at least one projection (5) which protrudes with respect to the charge carrier emission window (4) in the insertion direction and which is suitable for absorbing interfering radiation (8), and wherein the medium line (7) is capable of having a medium (9) flow through it which is capable of being discharged through the medium emission opening (12) at least into the region of the charge carriers issuing out of the charge carrier emission window (4), wherein the medium (9) is suitable for changing the dimension of a charge carrier cloud (6) formed by the issuing charge carriers, wherein
- the projection (5) protruding relative to the charge carrier emission window (4) runs at least in sections along a circumference of the treatment device (1),
- the projection (5) extends beyond the charge carrier emission window (4), is arranged in the vicinity of the charge carrier emission window (4) and the projection (5) extends from a plane formed by the charge carrier emission window (4) at least also in the direction of the emerging charge carrier beam.

2. An apparatus (1) for sterilizing an inner wall of containers (2) according to claim 1,
**characterized in that**
the medium line (7) has a curved shape at least locally, wherein the medium line (7) is preferably curved in such a way that the medium (9) capable of being conveyed in it is capable of being discharged through the medium emission opening (12) in the direction of the charge carrier cloud (6), preferably with a component in a direction opposed, and in a particularly preferred manner in a direction opposed to an acceleration direction (R) of the charge carriers.

3. An apparatus (1) for sterilizing an inner wall of containers (2) according to at least one of the preceding claims,
**characterized in that**
the medium line (7) is connected at least locally to the treatment device (1), is preferably integrated in the latter and is movable jointly with the treatment device (1).

4. A plant for the treatment of an inner wall of containers (2)
**characterized in that**
the plant has a plurality of apparatuses (1) for the sterilization of containers (2), according to at least one of the preceding claims.

5. A plant for the treatment of an inner wall of containers (2) according to claim 4,
**characterized in that**
the plant has an insertion device by means of which the treatment device (1) is capable of being introduced into the interior of a container (2), wherein the container (2) and the treatment device (1) are movable relative with respect to each other, preferably in a longitudinal direction of the container (2).

6. A method of sterilizing an inner wall of containers (2) by means of accelerated charge carriers, wherein the charge carriers are produced in a charge carrier source and are accelerated with an acceleration device in the direction of a charge carrier emission window (4), wherein the charge carrier emission window (4) is arranged on a treatment device (1) which is introduced through an opening into the container (2) along an insertion direction, in order to act upon an inner wall of the container (2) with the charge carriers issuing from the charge carrier emission window (4), wherein the treatment device (1) has a medium line (7) with a medium emission opening (12), the medium emission opening (12) of which is preferably capable of being introduced through the opening into the container (2),
**characterized in that**
the treatment device (1) has at least one projection (5) which protrudes with respect to the charge carrier emission window (4) in the insertion direction and which can absorb radiation (8), and wherein the medium line (7) is capable of having a medium (9) flow through it which is capable of being discharged through the medium emission opening (12) at least into the region of the charge carriers issuing out of the charge carrier emission window (4), wherein the medium (9) changes the dimension of a charge carrier cloud (6) formed by the issuing charge carriers, wherein
- the projection (5) protruding relative to the charge carrier emission window (4) runs at least in sections along a circumference of the treatment device (1),
- the projection (5) extends beyond the charge carrier emission window (4), is arranged in the vicinity of the charge carrier emission window (4) and the projection (5) extends from a plane formed by the charge carrier emission window (4) at least also in the direction of the emerging charge carrier beam.

## Revendications

1. Dispositif (1) de stérilisation d'une paroi interne de conteneurs (2) au moyen de porteurs de charge accélérés avec une source de porteurs de charge pour produire les porteurs de charge, doté d'un équipement d'accélération via lequel des porteurs de charge peuvent être accélérés en direction d'une fenêtre d'émission de porteurs de charge (4), dans lequel la fenêtre d'émission de porteurs de charge (4) étant agencée contre un équipement de traitement (1), lequel peut être introduit dans une direction d'introduction à travers un orifice dans le conteneur (2) pour solliciter une paroi interne du conteneur (2) avec les porteurs de charge sortant de la fenêtre d'émission de porteurs de charge (4), dans lequel l'équipement de traitement (1) présente une conduite pour substances (7) avec un orifice d'émission de substances (12), dont l'orifice d'émission de substances (12) peut être introduit à travers l'orifice dans le conteneur (2),
**caractérisé en ce que**
l'équipement de traitement (1) présente au moins une saillie (5) dépassant dans une direction d'introduction par rapport à la fenêtre d'émission de porteurs de charge (4), ladite saillie est adaptée pour absorber un rayonnement d'interférence (8) et dans lequel une substance (9) peut être amenée à travers la conduite pour substances (7), ladite substance peut être distribuée à travers l'orifice d'émission de substances (12) au moins dans la zone des porteurs de charge émettant de la fenêtre d'émission de porteurs de charge (4), dans lequel la substance (9) est adaptée pour modifier la dimension d'un nuage de porteurs de charge (6) formé par les porteurs de charge déjà émis, dans lequel
- la saillie (5) dépassant par rapport à la fenêtre d'émission de porteurs de charge (4) se déploie au moins partiellement le long d'une périphérie de l'équipement de traitement (1),
- la saillie (5), s'étendant au-delà de la fenêtre d'émission de porteurs de charge (4), est agencée à proximité de la fenêtre d'émission de porteurs de charge (4), et la saillie (5) s'étend depuis un plan formé par la fenêtre d'émission de porteurs de charge (4) au moins aussi en direction du rayon de porteur de charge émi.

2. Dispositif (1) de stérilisation d'une paroi interne de conteneurs (2) selon la revendication 1,
**caractérisé en ce que**
la conduite pour substances (7) présente au moins partiellement une forme incurvée, dans lequel la conduite pour substances (7) est de préférence incurvée de telle sorte que la substance (9) pouvant y être amené peut être distribué à travers l'orifice d'émission de substances (12) en direction du nuage de porteurs de charge (6), de préférence avec une composante allant à l'opposé, particulièrement de préférence à l'opposé d'une direction d'accélération (R) des porteurs de charge.

3. Dispositif (1) de stérilisation d'une paroi interne de conteneurs (2) selon au moins une des revendications précédentes,
**caractérisé en ce que**
la conduite pour substances (7) est raccordée au moins partiellement à l'équipement de traitement (1), de préférence intégrée à celle-ci et conjointement déplaçable avec l'équipement de traitement (1).

4. Installation de traitement d'une paroi interne de conteneurs (2),
**caractérisée en ce que**
l'installation présente une pluralité de dispositifs (1) de stérilisation de conteneurs (2) selon au moins une des revendications précédentes.

5. Installation de traitement d'une paroi interne de conteneurs (2) selon la revendication 4,
**caractérisée en ce que**
l'installation présente un équipement d'introduction au moyen duquel l'équipement de traitement (1) peut être introduit à l'intérieur d'un conteneur (2), dans laquelle le conteneur (2) et l'équipement de traitement (1) sont déplaçables relativement l'un de l'autre, de préférence dans une direction longitudinale du conteneur (2).

6. Procédé de stérilisation d'une paroi interne de conteneurs (2) au moyen de porteurs de charge accélérés, dans lequel des porteurs de charge étant produits dans une source de porteurs de charge et étant accélérés à l'aide d'un équipement d'accélération en direction d'une fenêtre d'émission de porteurs de charge (4), dans lequel la fenêtre d'émission de porteurs de charge (4) étant agencée contre un équipement de traitement (1), lequel est introduit dans une direction d'introduction à travers l'orifice dans le conteneur (2) pour solliciter une paroi interne du conteneur (2) avec les porteurs de charge émettant de la fenêtre d'émission de porteurs de charge (4), dans lequel l'équipement de traitement (1) présente une conduite pour substances (7) avec un orifice d'émission de substances (12), dont l'orifice d'émission de substances (12) peut être introduit à travers l'orifice dans le conteneur (2),
**caractérisé en ce que**
l'équipement de traitement (1) présente au moins une saillie (5) dépassant dans une direction d'introduction par rapport à la fenêtre d'émission de porteurs de charge (4), ladite saillie peut absorber un rayonnement d'interférence (8) et dans lequel une substance (9) peut être amenée à travers la conduite pour substances (7), ladite substance peut être distribuée à travers l'orifice d'émission de substances (12) au moins dans la zone des porteurs de charge sortant de la fenêtre d'émission de porteurs de charge (4), dans lequel la substance (9) modifie la dimension d'un nuage de porteurs de charge (6) formé par les porteurs de charge déjà émis, dans lequel
- la saillie (5) dépassant par rapport à la fenêtre d'émission de porteurs de charge (4) se déploie au moins partiellement le long d'une périphérie de l'équipement de traitement (1),
- la saillie (5), s'étendant au-delà de la fenêtre d'émission de porteurs de charge (4), est agencée à proximité de la fenêtre d'émission de porteurs de charge (4), et la saillie (5) s'étend depuis un plan formé par la fenêtre d'émission de porteurs de charge (4) au moins aussi en direction du rayon de porteur de charge émi.
